# EUROPÄISCHE PATENTSCHRIFT

(11) **EP 1 216 095 B1**
(45) Veröffentlichungstag und Bekanntmachung des Hinweises auf die Patenterteilung: **19.05.2004**
(21) Anmeldenummer: 00966020.0
(22) Anmeldetag: 15.09.2000
(51) Int. Cl.: B01J 23/745, B01J 37/18

(54) **VERFAHREN ZUR AKTIVIERUNG VON PASSIVIERTEM EISEN**
METHOD FOR ACTIVATING PASSIVATED IRON
PROCEDES POUR ACTIVER DU FER PASSIVE

(30) Priorität: 01.10.1999 DE 19947508
(43) Veröffentlichungstag der Anmeldung: 26.06.2002
(73) Patentinhaber: BASF AKTIENGESELLSCHAFT, 67056 Ludwigshafen (DE)
(72) Erfinder: OHLBACH, Frank, 69221 Dossenheim (DE); FISCHER, Rolf, 69121 Heidelberg (DE); LUYKEN, Hermann, 67069 Ludwigshafen (DE); MELDER, Johann-Peter, 67459 Böhl-Iggelheim (DE); MERGER, Martin, 67227 Frankenthal (DE); ANSMANN, Andreas, 69168 Wiesloch (DE); BASSLER, Peter, 68519 Viernheim (DE)
(86) Internationale Anmeldenummer: PCT/EP2000/009020
(87) Internationale Veröffentlichungsnummer: WO 2001/024925

(56) Entgegenhaltungen:
- EP-A- 0 402 727
- DE-A- 19 742 221
- US-A- 3 758 584
- US-A- 4 064 172

## Beschreibung

Die vorliegende Erfindung betrifft ein Verfahren zur Aktivierung von passiviertem, nach der Aktivierung als katalytisch aktive Komponente geeignetem Eisen mit Wasserstoff bei erhöhter Temperatur und erhöhtem Druck, dadurch gekennzeichnet, daß man die Aktivierung in Gegenwart eines Nitrils und bei einer Temperatur im Bereich zwischen 20 und 180°C durchführt.

Ganz oder weitgehend aus elementarem Eisen bestehende Katalysatoren besitzen große Bedeutung, z. B. für die Synthese von Ammoniak aus Stickstoff und Wasserstoff, für die Herstellung von Kohlenwasserstoffen aus Synthesegas (Fischer-Tropsch-Synthese) und für die Hydrierung von Nitrilen zu Aminen (J.W. Geus, Applied Catalysis 25, Seiten 313 bis 333 (1986).

Derartige Katalysatoren werden meist durch Reduktion von Eisenoxiden mit Wasserstoff hergestellt. Hierzu wird das Eisenoxid bei hohen Temperaturen im Wasserstoffstrom reduziert, wobei der Oxidsauerstoff zu Wasser umgesetzt und in dieser Form ausgetragen wird.

In DP-PS 855.263 wird die Reduktion von aufgeschmolzenem und anschließend zerkleinertem Eisenoxid bei 400°C im Wasserstoffstrom beschrieben.

In J. Mater. Sci. Lett. 8 (8) (1989), Seiten 895 bis 898 wird experimentell festgestellt, daß die vollständige Reduktion von Eisenoxiden zu Eisen im Wasserstoffstrom nur bei Temperaturen oberhalb von 400°C, bzw. die Reduktion von dotierten Eisenoxiden, wie sie für die Ammoniak-Synthese Verwendung finden, nur oberhalb von 500 °C erreicht werden kann.

Aus US 3,758,584, Spalte 1, Zeilen 47 bis 65, geht hervor, daß die Reduktion der Eisenoxide bei 300 bis 600 °C in Gegenwart von 0,01 bis 10 Volumenprozent Ammoniak durchgeführt wird. Bevorzugt ist eine Temperatur von 350 bis 420 °C, wobei der Wasserstoff 0,25 bis 3 Volumenprozent Ammoniak enthält (Spalte 2, Zeilen 12 bis 18). Derartige Eisenkatalysatoren werden z. B. für die Hydrierung von Adipodinitril zu Hexamethylendiamin verwendet.

Die Reduktion kann gemäß US 4,480,051 auch dreistufig erfolgen, indem man in einem ersten Schritt das Eisenoxid mit Wasserstoff oder Gemischen aus Wasserstoff und Ammoniak reduziert, dann das gebildete elementare Eisen in einem zweiten Schritt mit einem Sauerstoff enthaltenden Gas behandelt und anschließend in einem dritten Schritt die Reduktion analog zum ersten Schritt wiederholt.

Der nach den genannten Verfahren hergestellte reduzierte Eisenkatalysator ist pyrophor. Wurde die Reduktion des Eisenoxids direkt im für die spätere Umsetzung vorgesehenen Synthesereaktor durchgeführt, so kann der Katalysator anschließend für die vorgesehene chemische Umsetzung verwendet werden. Die Reduktion im Synthesereaktor besitzt jedoch Nachteile: Da die Reduktion zusammen mit Aufheizen und Abkühlen viele Stunden dauert, steht der Reaktor in dieser Zeit nicht für die Produktion zur Verfügung. Außerdem kann die Reduktionstemperatur deutlich über der späteren Synthesetemperatur liegen. Dies bedeutet apparativen Mehraufwand am Reaktor, der ausschließlich für die Reduktion benötigt wird.

Daher kann es vorteilhaft sein, das Eisenoxid außerhalb des vorgesehenen Synthesereaktors zu reduzieren. Für den Transport zum Synthesereaktor und für das Einfüllen muß der pyrophore Katalysator jedoch durch Behandlung mit Luft passiviert werden.

Diese Passivierung kann nach WO 98/11.059 bei Temperaturen im Bereich von 20 bis 80°C, vorzugsweise 25 bis 60°C, z. B. mit Stickstoff-Sauerstoff-Mischungen erfolgen. Die Aktivierung derartiger Katalysatoren ("reduziert/passiviert") nimmt man dann im Synthesereaktor bei einer Temperatur im Bereich von 180 bis 500°C, vorzugsweise von 200 bis 400 °C in einer wasserstoffhaltigen Atmosphäre vor.

Unter Aktivierung wird im Sinne der vorliegenden Erfindung die Umwandlung eines reduziert/passivierten Eisens in eine katalytisch aktive Form verstanden.

Nachteilig ist, daß auch für die Nachaktivierung im Reaktor hohe Temperaturen im Bereich von 200 bis 400°C erforderlich sind. Dies führt zu erheblichen Mehrkosten wegen des erhöhten apparativen Aufwands (Vorheizer, Kreisgasverdichter, Reaktormaterial usw.). Außerdem ist der Zeitbedarf für die Aktivierung zwar niedriger als bei der anfänglichen Reduktion des Eisenoxids, aber immer noch hoch. So wird in A. V. Slack, G. R. James: Ammonia, Part II, Marcel Dekker Inc., 1977, Seiten 113 bis 114 die Vorgehensweise bei der Aktivierung eines passivierten Eisens, das bei der Ammoniaksynthese Verwendung findet, beschrieben. Die Aktivierung findet bei 300 bis 480°C statt und dauert ungefähr 17 Stunden, wozu noch einmal die gleiche Zeit für das Aufheizen des Reaktors hinzugezählt werden muß.

DE-A-3,524,330 beschreibt die Aktivierung von passiviertem Eisen in Gegenwart eines Redox-Systems, z.B. Keton/Alkohol, bei Temperaturen von etwa 200°C. Nachteilig hierbei sind die hohe Temperatur und der erhebliche Zeitaufwand für die Aktivierung.

Der vorliegenden Erfindung lag daher die Aufgabe zugrunde, ein Verfahren zur Verfügung zu stellen, das die Aktivierung von passiviertem, nach der Aktivierung als katalytisch aktive Komponente geeignetem Eisen mit Wasserstoff bei erhöhter Temperatur und erhöhtem Druck auf technisch einfache und wirtschaftliche Weise ermöglicht unter Vermeidung der genannten Nachteile.

Demgemäß wurde das eingangs definierte Verfahren gefunden.

Das in dem erfindungsgemäßen Verfahren eingesetzte passivierte, nach der Aktivierung als katalytisch aktive Komponente geeignete Eisen kann nach an sich bekannten Verfahren erhalten werden.

So können als Vorläufer eines solchen Eisens Eisenoxide, Eisenhydroxide, Eisenoxyhydroxide oder deren Gemische eingesetzt werden (Komponente a). Als solche kommen beispielsweise Eisen-(III)-oxid, Eisen-(II, III)-oxid, Eisen-(II)-oxid, Eisen-(II)-hydroxid, Eisen-(III)-hydroxid oder Eisenoxyhydroxid wie FeOOH in Betracht. Verwendet werden können synthetisch hergestellte oder natürlich vorkommende Eisenoxide, Eisenhydroxide oder Eisenoxyhydroxide, wie Magneteisenstein (Magnetit), der im Idealfall mit Fe₃O₄ beschrieben werden kann, Brauneisenstein, der im Idealfall mit Fe₂O₃ x H₂O beschrieben werden kann, oder Roteisenstein (Hämatit), der im Idealfall mit Fe₂O₃ beschrieben werden kann.

Solche Verbindungen können zur Herstellung von Eisen enthaltenden Trägerkatalysatoren, vorzugsweise zur Herstellung von Eisen enthaltenden Vollkatalysatoren eingesetzt werden.

Als Vorläufer eines solchen Eisens können als Komponente a) gut wasserlösliche Salze des Eisens, wie Nitrate, Chloride, Acetate, Formiate oder Sulfate, vorzugsweise Nitrate, oder deren Gemische, sowie Gemische solcher Salze mit den bereits genannten Eisenoxiden, Eisenhydroxiden oder Eisenoxyhydroxiden eingesetzt werden.

Solche Verbindungen können zur Herstellung von Eisen enthaltenden Vollkatalysatoren, vorzugsweise zur Herstellung von Eisen enthaltenden Trägerkatalysatoren eingesetzt werden.

Das in dem erfindungsgemäßen Verfahren eingesetzte passivierte, nach der Aktivierung als katalytisch aktive Komponente geeignete Eisen kann weitere Komponenten, wie Promotoren, enthalten.

Als solche kommen vorteilhaft einzelne oder mehrere der folgenden Elemente oder Verbindungen auf Basis der folgenden Elemente oder deren Gemische in Betracht (Komponente (b)):
Palladium, Cobalt, Ruthenium, Rhodium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, Silizium, Zirkonium, Vanadium, Titan.

Als weitere Komponente (Komponente (c)) kommen vorteilhaft einzelne oder mehrere Verbindungen auf Basis eines oder mehrerer Alkali- oder Erdalkalimetalle in Betracht.

Zur Herstellung des passivierten, nach der Aktivierung als katalytisch aktive Komponente geeigneten Eisens kann der Vorläufer der Komponente (a) bereits die Komponente (b) oder deren Vorläufer teilweise oder vollständig enthalten. Ebenso kann zur Herstellung des passivierten, nach der Aktivierung als katalytisch aktive Komponente geeigneten Eisens der Vorläufer der Komponente (a) bereits die Komponente (c) oder deren Vorläufer teilweise oder vollständig enthalten.

Als Vorläufer der Komponente (b) kommen vorzugsweise gut wasserlösliche Salze oder Komplexsalze der genannten Elemente in Betracht, wie Nitrate, Chloride, Acetate, Formiate, Sulfate, vorzugsweise Nitrate.

Als Vorläufer der Komponente (c) kommen vorzugsweise gut wasserlösliche Salze oder Komplexsalze der genannten Elemente in Betracht, wie Hydroxide, Carbonate, Nitrate, Chloride, Acetate, Formiate, Sulfate, vorzugsweise Hydroxide und Carbonate.

Katalysatorvorläufer, die passiviertes, nach der Aktivierung als katalytisch aktive Komponente geeignetes Eisen und gegebenenfalls Komponente (b) oder Komponente (c) oder die Komponenten (b) und (c) enthalten, können als Vorläufer für Vollkatalysatoren oder Trägerkatalysatoren dienen.

Solche Trägerkatalysatoren können an sich bekannte Trägermaterialien enthalten, vorzugsweise Aluminiumoxid, Siliziumoxid, Alumosilikate, Lanthanoxid, Titandioxid, Zirkondioxid, Magnesiumoxid, Zinkoxid, Zeolithe oder Aktivkohle sowie deren Mischungen.

Die Herstellung der Katalysatorvorläufer kann in der Regel derart erfolgen, daß man Vorläufer der Komponente (a) gewünschtenfalls zusammen mit Vorläufern der Komponente (b) und gewünschtenfalls mit Vorläufern der Komponenten (c) in Abwesenheit, oder im Falle von Trägerkatalysatoren in Gegenwart, von Trägermaterialien ausfällt, gewünschtenfalls den so erhaltenen Katalysatorvorläufer zu Strängen oder Tabletten verarbeitet, trocknet und anschließend calciniert.

Die Fällung kann im allgemeinen aus wäßrigen Lösungen in an sich bekannter Weise erfolgen, beispielsweise durch Zugabe von Fällungsreagenzien, durch Änderung des pH-Werts oder durch Änderung der Temperatur.

Trägerkatalysatoren sind im allgemeinen auch erhältlich, indem man ein Trägermaterial mit einer Lösung der Komponente (a), gewünschtenfalls Komponente (b) und gewünschtenfalls Komponente (c) tränkt, wobei man die einzelnen Komponenten gleichzeitig oder nacheinander zugeben kann, oder indem man die Komponenten (a), gewünschtenfalls Komponente (b) und gewünschtenfalls Komponente (c) auf ein Trägermaterial nach an sich bekannten Methoden aufsprüht.

In an sich üblicher Weise kann man die so erhaltene Katalysator-Vorläufer-Masse im allgemeinen bei Temperaturen im Bereich von 80 bis 150, vorzugsweise von 80 bis 120°C vortrocknen.

Anschließend folgt in der Regel eine Calcination. Das Calcinieren nimmt man üblicherweise bei Temperaturen im Bereich von 150 bis 500°C, vorzugsweise von 200 bis 450°C in einem Gasstrom, beispielsweise aus Luft oder Stickstoff, vor.

Die Katalysatorvorläufer, enthaltend passiviertes, nach der Aktivierung als katalytisch aktive Komponente geeignetes Eisen, können auch durch Aufschmelzen von eisenhaltigen Oxiden, Hydroxiden oder Oxidhydroxiden oder Gemischen solcher Verbindungen bei Temperaturen oberhalb von 1500°C erhalten werden. Diese eisenhaltigen Ausgangsverbindungen können Komponente (b) oder Komponente (c) bereits enthalten. Es ist ebenso möglich, gewünschtenfalls Komponente (b) oder Komponente (c) oder Komponenten (b) und (c) zuzusetzen.

Nach dem Calcinieren setzt man in der Regel die erhaltene Katalysator-Vorläufer-Masse einer reduzierenden Atmosphäre aus. Dazu setzt man vorzugsweise die Katalysator-Vorläufer-Masse bei einer Temperatur im Bereich von 200 bis 500°C, vorzugsweise von 250 bis 450°C einer Gasatmosphäre, enthaltend molekularen Wasserstoff und gewünschtenfalls weitere Gase, insbesondere Inertgase, wie Stickstoff, aus. Die Reaktionszeiten betragen im allgemeinen 2 bis 72 Stunden. Die Belastung des Katalysator-Vorläufers beträgt hierbei bevorzugt 100 bis 500 N1/1 Katalysator-Vorläufer x Stunde, für Ammoniaksynthese-Katalysator-Vorläufer 2.000 bis 10.000 N1/1 Katalysator-Vorläufer x Stunde.

Die Passivierung des metallisches Eisen enthaltenden Katalysator-Vorläufers erfolgt in der Regel bei 20 bis 100°C, vorzugsweise 25 bis 60°C. Dazu kann man vorteilhaft die Katalysator-Vorläufer in Kontakt mit einem oxidierend wirkenden Gas, vorzugsweise mit einem molekularen Sauerstoff enthaltenden Gas, insbesondere mit einer Mischung aus Stickstoff und Sauerstoff, bringen.

Zur Herstellung eines passivierten, nach der Aktivierung als katalytisch aktive Komponente geeigneten Eisen enthaltenen Katalysators wird erfindungsgemäß der Katalysator-Vorläufer mit Wasserstoff bei einer Temperatur im Bereich zwischen 20 und 180°C und erhöhtem Druck, in Gegenwart eines Nitrils aktiviert.

Der Wasserstoff kann als reines Gas oder als Gas, enthaltend weitere Bestandteile, wie Inertgas, beispielsweise Stickstoff oder Argon, insbesondere Stickstoff, eingesetzt werden.

Die Aktivierung des Katalysator-Vorläufers kann man vorteilhaft in dem Reaktor vornehmen, in dem der Katalysator nach der Aktivierung eingesetzt wird. Der Katalysator-Vorläufer kann dazu als Festbett in dem Reaktor angeordnet oder in dem Reaktor in einem Lösungsmittel suspendiert werden.

Als Nitrile können einzeln oder im Gemisch prinzipiell alle Nitrile, also organischen Verbindungen, die mindestens eine, vorzugsweise mehrere, wie zwei, drei oder vier, in dem erfindungsgemäßen Verfahren reaktive Nitril-Gruppen aufweisen, eingesetzt werden, wie aromatische Nitrile, also Nitrile, bei denen die Nitril-Gruppe mit einem Aromaten oder Arylaliphaten direkt verknüpft ist, beispielsweise Benzonitril, o-Aminobenzonitril oder Phthalodinitril, oder aliphatische Nitrile, also Nitrile, bei denen die Nitril-Gruppe mit einem aliphatischen System oder dem aliphatischen Teil eines Arylaliphaten direkt verknüpft ist.

Die aromatischen oder aliphatischen Nitrile können eine oder mehrere, wie zwei, drei oder vier weitere gleiche oder unterschiedliche funktionelle Gruppen, wie Amino-Gruppen oder Ester-Gruppen, tragen.

Als aliphatische Nitrile sind solche mit 1 bis 20 C-Atomen geeignet, gerechnet ohne die Nitril-Gruppe oder Nitril-Gruppen, wobei das aliphatische System linear, verzweigt oder cyclisch sein kann, wie lineare Nitrile mit einer Nitril-Gruppe, beispielsweise Acetonitril oder Propionitril, cyclische Nitrile mit einer Nitril-Gruppe, beispielsweise Cyclohexylnitril, lineare Nitrile mit einer Nitril-Gruppe und einer weiteren funktionellen Gruppe, beispielsweise Cyanoessigsäureester oder 6-Aminocapronitril, wie lineare Nitrile mit zwei Nitril-Gruppen, beispielsweise 2-Methylglutaronitril, Adipodinitril oder Bernsteinsäuredinitril.

Selbstverständlich können auch Gemische mehrerer Nitrile eingesetzt werden.

Die Herstellung solcher Nitrile ist an sich bekannt.

Bei einer Anordnung des Katalysator-Vorläufers als Festbett in einem Reaktor kann man die Aktivierung kontinuierlich oder diskontinuierlich in Riesel- oder Sumpffahrweise durchführen. Die Temperatur liegt erfindungsgemäß im Bereich zwischen 20 und 180°C, insbesondere 30 bis 150°C. Der Druck sollte vorteilhaft im Bereich zwischen 2 und 40 MPa, insbesondere 3 bis 30 MPa liegen.

Bevorzugt kann man die Aktivierung in Gegenwart eines anorganischen Lösungsmittel, wie Ammoniak, oder eines organischen Lösungsmittels, wie einem Amin, Diamin oder Triamin, vorzugsweise mit 1 bis 6 C-Atomen, beispielsweise Trimethylamin, Triethylamin, Tri-n-propylamin, Tri-i-propylamin, Tributylamin, wie einem Alkohol, beispielsweise Methanol, Ethanol, n-Propanol, i-Propanol, n-Butanol, i-Butanol, s-Butanol, bevorzugt Ammoniak durchführen.

In einer bevorzugten Ausführungsform kann man 1 bis 10 g, vorzugsweise 2 bis 6 g Lösungsmittel, insbesondere Ammoniak, pro g Nitril einsetzen.

Vorteilhaft kommt eine Belastung des Katalysator-Vorläufers im Bereich von 0,1 bis 2,0 kg, insbesondere 0,3 bis 1,0 kg Nitril/1 x h in Betracht.

Beim Einsatz des Katalysator-Vorläufers als Suspension in einem Lösungsmittel kann man die Aktivierung diskontinuierlich oder bevorzugt kontinuierlich inbesondere in der Flüssigphase durchführen. Die Temperatur sollte vorteilhaft im Bereich zwischen 20 und 180°C, vorzugsweise 40 und 150°C, insbesondere 50 bis 100°C liegen. Der Druck sollte vorteilhaft im Bereich zwischen 2 und 40 MPa, vorzugsweise 2 und 30 MPa, besonders bevorzugt 3 bis 30 MPa, insbesondere 4 bis 9 MPa liegen. Die Verweilzeit sollte vorteilhaft im Bereich zwischen 50 und 275 min, vorzugsweise 70 bis 200 min liegen.

Bevorzugt kann man die Aktivierung in Gegenwart eines anorganischen Lösungsmittel, wie Ammoniak, oder eines organischen Lösungsmittels, wie einem Amin, Diamin oder Triamin, vorzugsweise mit 1 bis 6 C-Atomen, beispielsweise Trimethylamin, Triethylamin, Tri-n-propylamin, Tri-i-propylamin, Tributylamin, wie einem Alkohol, beispielsweise Methanol, Ethanol, n-Propanol, i-Propanol, n-Butanol, i-Butanol, s-Butanol, bevorzugt Ammoniak durchführen.

In einer bevorzugten Ausführungsform wählt man eine Nitrilkonzentration im Bereich von 10 bis 90 Gew.-%, vorzugsweise 30 bis 80 Gew.-%, insbesondere 40 bis 70 Gew.-%, bezogen auf die Summe aus Nitril und Lösungsmittel.

In einer bevorzugten Ausführungsform liegt die Menge an Katalysator-Vorläufer, bezogen auf die Menge an Nitril, im Bereich von 1 bis 50 Gew.-%, insbesondere 5 bis 20 Gew.-%.

Die in dem erfindungsgemäßen Verfahren eingesetzten Nitrile werden nach der Aktivierung in der Regel zum Teil als Amine, zum Teil als Carbonsäureamide erhalten.

Der Verlauf der Aktivierung kann in an sich bekannter Weise, beispielsweise durch gaschromatographische Analyse des Reaktionsaustrags im Falle einer kontinuierlichen Reaktionsführung oder gaschromatographische Analyse von Proben der Reaktionsmischung im Falle einer diskontinuierlichen Reaktionsführung, verfolgt werden.

Besonders vorteilhaftes, nach dem erfindungsgemäßen Verfahren erhältliches katalytisch aktives Eisen weist einen Gehalt von 0 bis 25 Gew.-% der Komponente (b), bezogen auf Komponente (a), und einen Gehalt von 0 bis 10 Gew.-% der Komponente (c), bezogen auf Komponente (a), auf.

Die optimalen Parameter zur Herstellung der gewünschten Zusammensetzung können in wenigen Vorversuchen einfach ermittelt werden.

Das nach dem erfindungsgemäßen Verfahren erhältliche Eisen kann in zahlreichen chemischen Verfahren, beispielsweise der Synthese von Ammoniak aus Stickstoff und Wasserstoff, der Herstellung von Kohlenwasserstoffen aus Synthesegas (Fischer-Tropsch-Synthese) oder bei Hydrierungen, wie der Hydrierung von Nitrilen, insbesondere alpha,omega-Dinitrilen zu alpha,omega-Aminonitrilen oder alpha,omega-Diaminen, als katalytisch aktive Komponente in Heterogenkatalysatoren eingesetzt werden.

Bevorzugte Verwendung der Heterogenkatalysatoren ist dabei die Hydrierung von Adipodinitril zu Hexamethylendiamin und die partielle Hydrierung von Adipodinitril zu 6-Aminocapronitril und Hexamethylendiamin.

Wird der Katalysator für die Synthese von Ammoniak aus Stickstoff und Wasserstoff oder für die Herstellung von Kohlenwasserstoffen aus Synthesegas (Fischer-Tropsch-Synthese) verwendet, so kann der Heterogenkatalysator vorteilhaft nach der Aktivierung mit Lösungsmittel, wie Alkoholen, Kohlenwasserstoffen, Aminen, Ammoniak oder Ether gewaschen und gegebenenfalls getrocknet werden.

Wird der Katalysator für die Hydrierung von Nitrilen verwendet, so kann das an dem aktivierten Katalysator umzusetzende Nitril vorteilhaft während der Aktivierung in dem erfindungsgemäßen Verfahren eingesetzt werden.

### Beispiel 1

Alle %-Angaben in dem Beispiel betreffen, sofern nicht anders angegeben, Gew.-%.

### a) Aktivierung von reduziert passiviertem Eisen

Der Katalysator-Vorläufer wurde hergestellt durch Aufschmelzen eines Magnetiterzes oberhalb von 1550°C an Luft. Das verwendete Magnetiterz hatte folgende Zusammensetzung:
72 % Fe, 0,08 % Al, 0,03 % Ca, 0,05 % Mg, 0,12 % Si, 0,01 % Ti, 0,17 % Mn, Rest Sauerstoff.

Der abgekühlte Schmelzblock wurde im Backenbrecher zerkleinert. Eine Siebfraktion von 1,5 bis 3 mm wurde ausgesiebt und im Wasserstoff/Stickstoffstrom binnen 72 Stunden bei 450°C reduziert. Nach Abkühlen unter Stickstoff wurde der Katalysator mit einem Gemisch aus 1 % Luft mit Stickstoff binnen 24 Stunden passiviert, wobei darauf geachtet wurde, daß die Temperatur des Hot-Spots in der Schüttung 45°C nicht überstieg.

In einem 270-ml-Autoklaven wurden 80 g reduziert/passivierter Katalysator-Splitt in einem Drahtkorb eingebaut und der Autoklav verschlossen. Anschließend wurden 69 g Ammoniak und 34,5 g ACN eingefüllt, der Autoklav auf 110°C erwärmt und über einen Begasungsrührer bei 2000 U/min mit Wasserstoff auf 250*10⁵ Pa Gesamtdruck aufgepreßt. Nach etwa einer Stunde begann die Wasserstoffaufnahme, wobei die Aufnahmegeschwindigkeit so lange nahezu linear mit der Versuchsdauer anstieg, bis die Nitrile vollständig umgesetzt waren. Während der Reaktion wurden Proben gezogen und es zeigte sich, daß ACN vollständig zu HMD und ACSA umgesetzt wurde (Tab. 1).
ACN = 6-Aminocapronitril
HMD = Hexamethylendiamin
ACSA = 6-Aminocapronsäureamid
ADN = Adipodinitril

**Tab. 1**

| Rkt.-zeit (min) | ACN (F1-%) | HMD (F1-%) | ACSA (F1-%) |
|---|---|---|---|
| 70 | 96,22 | 1,10 | 2,10 |
| 135 | 61,02 | 30,93 | 6,92 |
| 195 | 2,95 | 86,89 | 8,80 |
| 255 | 0,54 | 89,36 | 8,76 |
| 360 | 0,17 | 89,73 | 8,73 |

Tabelle 1 zeigt, daß das gebildete Wasser mit ACN zu ACSA reagiert.

### b) Hydrierung von Adipodinitril zu Hexamethylendiamin

Der nach 1 a) durch Aktivierung von reduziert-passiviertem Eisenkatalysator hergestellte Katalysator wurde im selben Reaktor für die Hydrierung von Adipodinitril zu Hexamethylendiamin verwendet. Der Reaktor wurde im Gegensatz zu 1a) kontinuierlich betrieben. Hydriert wurde bei 135 °C, 250*10⁵ Pa und einer Katalysatorbelastung von 1,0 kg ADN/1 Kat. x h. Ein Gemisch aus Ammoniak und ADN im Gewichtsverhältnis von 2 : 1 wurde dem Reaktor kontinuierlich zugeführt. Während eines Zeitraums von 100 Stunden wurden Proben entnommen und gaschromatographisch analysiert. Dabei ergab sich bei vollständigem ADN-Umsatz eine Hexamethylendiamin-Ausbeute von 99 %.

## Patentansprüche

1. Verfahren zur Aktivierung von passiviertem, nach der Aktivierung als katalytisch aktive Komponente geeignetem Eisen mit Wasserstoff bei erhöhtem Druck, **dadurch gekennzeichnet, daß** man die Aktivierung in Gegenwart eines Nitrils und bei einer Temperatur im Bereich zwischen 20 und 180°C durchführt.

2. Verfahren nach Anspruch 1, wobei man die Aktivierung bei einem Druck im Bereich von 2 bis 40 MPa durchführt.

3. Verfahren nach Anspruch 1 oder 2, wobei man als Nitril ein aliphatisches Nitril einsetzt.

4. Verfahren nach den Ansprüchen 1 bis 3, wobei man als Nitril eine Verbindung einsetzt ausgewählt aus der Gruppe bestehend aus Adipodinitril und 6-Aminocapronitril.

## Claims

1. A process for high pressure hydrogen activation of passivated iron useful as catalytically active component after said activation, which comprises effecting said activation in the presence of a nitrile at from 20 to 180°C.

2. A process as claimed in claim 1, wherein said activation is effected at from 2 to 40 MPa.

3. A process as claimed in claim 1 or 2, wherein the nitrile used is an aliphatic nitrile.

4. A process as claimed in any of claims 1 to 3, wherein the nitrile used is a compound selected from the group consisting of adiponitrile and 6-aminocapronitrile.

## Revendications

1. Procédé d'activation de fer passivé, approprié comme composant catalytiquement actif après l'activation, par de l'hydrogène à une pression élevée, **caractérisé en ce qu'**on effectue l'activation en présence d'un nitrile et à une température dans la gamme comprise entre 20 et 180°C.

2. Procédé suivant la revendication 1, dans lequel on effectue l'activation à une pression de l'ordre de 2 à 40 MPa.

3. Procédé suivant l'une des revendications 1 et 2, dans lequel on met en oeuvre un nitrile aliphatique en qualité de nitrile.

4. Procédé suivant l'une des revendications 1 à 3, dans lequel on met en oeuvre, en qualité de nitrile, un composé choisi parmi le groupe constitué de l'adipodinitrile et du 6-aminocapronitrile.
